# EUROPEAN PATENT APPLICATION

(11) **EP 2 042 605 A1**
(43) Date of publication of application: **01.04.2009**
(21) Application number: 08165069.9
(22) Date of filing: 25.09.2008
(51) Int. Cl.: C12N 15/867, C12N 9/22, A61K 48/00

(54) **Integration optimised viral vectors for gene therapy**

(30) Priority: 25.09.2007 EP 07117180
(71) Applicant: University of Veterinary Medicine Vienna, Research Institute for Virology and Biomedicine, 1210 Vienna (AT)
(72) Inventor: Indik, Stanislav, 1210 Vienna (AT)
(74) Representative: Fleuchaus, Andrea

(57) **Abstract**

The present invention relates to the area of retroviral vector optimisation. Particularly, to the optimisation and thereby improved control of retroviral integration. For this the present invention teaches and provides integration optimised viral vectors as well as methods and applications to use them.

SEQUENCE LISTING

<110> University of Veterinary Medicine Vienna

<120> Interation optimised viral vectors for gene therapy

<130> P11229EP01

<150> EP 07117180.5
<151> 2007-09-25

<160> 5

<170> PatentIn version 3.3

<210> 1
<211> 20
<212> DNA
<213> artificial sequence

<220>
<223> PCR Primer

<400> 1
ccaacgagaa gcgcgatcac 20

<210> 2
<211> 20
<212> DNA
<213> artificial sequence

<220>
<223> PCR-Pimer

<400> 2
cgtctccgct cgtcacttat 20

<210> 3
<211> 20
<212> DNA
<213> artificial sequence

<220>
<223>  PCR-Primer

<400>  3
ctcggcatgg acgagctgta                                                    20

<210>  4
<211>  22
<212>  DNA
<213>  artificial sequence

<220>
<223>  PCR Primer

<400>  4
gtaatacgac tcactatagg gc                                                 22

<210>  5
<211>  19
<212>  DNA
<213>  artificial sequence

<220>
<223>  linker

<400>  5
agggctccgc ttaagggac                                                     19

## Description

Based on integration site preferences, retroviruses can be placed into three groups. Viruses that comprise the first group, murine leukemia virus (MLV) and foamy virus (FV) integrate preferentially near transcription start sites. The second group, notably human immunodeficiency virus (HIV) and simian immunodeficiency virus (SIV), preferentially targets transcription units. Avian sarcoma-leukosis virus (ASLV) and human T-cell leukemia virus (HTLV), forming the third group, show little preference for any genomic feature.

We have previously shown that some human cells sustain MMTV infection, therefore we infected the susceptible human breast cell line, Hs578T, and, without introducing a species-specific bias, compared the MMTV integration profile to that of other retroviruses. Additionally, we infected mouse cell line, NMuMG, and thus we could compare the MMTV integration site selection in human and mouse cells.

In total, we examined 468 unique MMTV integration sites. Irrespective whether human or mouse cells were infected, no integration bias favoring transcription start sites was detected, a profile that is reminiscent of that of ASLV and HTLV. However, in contrast to ASLV and HTLV, not even a modest tendency in favor of integration within genes was observed. Similarly, repetitive sequences and genes that are frequently tagged by MMTV in mammary tumors were not preferentially targeted in cell culture neither in mouse nor in human cells, hence we conclude that MMTV displays the most random dispersion of integration sites among retroviruses determined so far.

### BACKGROUND

Integration of proviral DNA into the host cell genome is an essential step in retroviral replication. Determination of integration profiles of various retroviruses is relevant not only for a more detailed understanding of the early steps of retroviral replication but might also be beneficial for the development of drugs targeting the integration process or to create safer retroviral vectors for gene therapy.

We have analyzed integration targeting of mouse mammary tumor virus (MMTV) in human and murine genomes, respectively. The aim of this study was to compare the distribution of the *de novo* integration sites generated experimentally with other retroviruses for which similar studies have already been performed. Secondly, we also investigated the integration pattern of MMTV in cells from two different species and thus we were able to analyze whether there is any species-specif effect that would influence the integration targeting.

At present, most of the studies on retroviral integration sites have been conducted with retroviruses or retroviral vectors targeting human genome. There are only few reports investigating the distribution of integration sites in more than one species. For example, Barr and coworkers analyzed ASLV and HIV integration target preferences in the chicken genome and compared it with the distribution of the integration sites observed in the human genome. The distributions of *de novo* integration sites for both viruses were generally similar in both genomes, suggesting that any cellular factor(s) important for insertional targeting are likely conserved between chickens and humans (2).

So far, a number of retroviruses belonging to different genera have been analyzed with respect to their integration site selection (2, 6, 10, 12, 21, 24, 28, 29, 34, 42, 43). Interestingly, retroviruses belonging to diverse genera display different preferences for target site selection (12, 28). It also appears that the integration profile of various retroviruses can be predicted from the phylogenetic relationships of retroviral integrases (12). The lentiviruses, human immunodeficiency virus (HIV) as well as simian immunodeficiency virus (SIV), tend to integrate within genes or transcription units (2, 6, 21, 28, 34, 43). By contrast, a gammaretrovirus, Murine leukemia virus (MLV), that has been utilized in gene therapy clinical trials for over a decade, shows a bias in favor of integration near transcription start sites and CpG islands (43). A similar integration pattern was also demonstrated for a member of the spumavirus genus, foamy virus (FV) (42). The other retroviruses studied so far, avian sarcoma-leukosis virus (ASLV, alpharetrovirus) and human T-cell leukemia virus (HTLV, deltaretrovirus), show the most random distribution of integration sites to date (2, 12, 28, 29, 42).

To our knowledge, no work analyzing *de novo* insertional preferences of a Betaretrovirus, the remaining genus in the Orthoretrovirinae subfamily, has been published to date. In this study, we have analyzed the insertion site preferences of MMTV, a Betaretrovirus that is known to be associated with mammary adenocarcinomas and T-cell lymphomas in mice (3, 11, 13, 27). MMTV is believed to cause tumors, in most cases via insertional mutagenesis. Insertion of an MMTV provirus in the vicinity of a proto-oncogene leads to an inappropriate transcriptional activation of adjacent genes that cause oncogenic transformation of the infected cells (other mutagenic events resulting in cell transformation, such as promoter insertion or truncations, are less common) and their clonal expansion (9). MMTV-based insertional mutagenesis is one of the most efficient methods to identify previously unknown genes and potential gene pathways that are involved in mammary differentiation and neoplasia. For this reason, the determination of MMTV integration sites in mouse mammary tumors has attracted the interest of many researchers and a number of MMTV insertion sites have been mapped (7, 8, 15, 16, 26, 30-33, 35, 37, 39, 40). MMTV proviral sequences were often found near one of the *Wnt, Notch* or *Fgf* gene families. Although, these common insertion sites were determined in clonally expanded cells, in which the MMTV enhancer activated the nearby proto-oncogene promoter, it is still not clear whether MMTV shows a bias in favor of integration, for example, within genes or transcription start sites.

To determine whether the integration of MMTV proviruses shows any bias in favor of any genomic feature or whether the virus rather exhibits a more random integration pattern, we have cloned and sequenced 468 unique virus-host junction sequences from acutely infected cells. The integration sites were then analyzed and compared with the integration profile of the other retroviruses for which such an analysis has been already performed.

### DETAILED DESCRIPTION OF THE INVENTION

### Materials and Methods

MMTV-EGFP and MMTV(GR) virus particles were prepared as described previously (18). Briefly, the production of the viruses was induced by adding dexamethasone (10⁻⁶M DEX) to CGRES6 or GR producer cell lines 24h before virus harvest, Supernatants containing the viral particles were filtered through 0.45-µm filter and concentrated (MMTV-EGFP) by ultracentrifugation at 120 000 × g for 2h at 4°C. The viral particles were resuspended in 1/100 volume of fresh medium and used for infection of a human breast cell line, Hs578T, or a mouse mammary cell line, NMuMG. The Hs578T cells infected by MMTV(GR) were propagated in 10⁻⁶M DEX-containing medium for four weeks.

Integration sites were cloned by ligation-mediated PCR essentially as described previously (43). Briefly, DNA was harvested 48h after infection with MMTV-EGFP. DNA from the MMTV(GR)-infected Hs578T cells was harvested four weeks after the initial infection. The genomic DNA was digested with Msel and linker DNA was ligated to the digested ends.

Virus-host junction sequences were PCR amplified using one primer complementary to *EGFP* or to viral LTR sequence and the other primer complementary to the linker (43). Nested PCR was performed with another primer pair, the resulting products were cloned into pCR2.1 vector (Invitrogen, CA) and sequenced. The primers used for this study were: EGFP1, 5'-CCA ACG AGA AGC GCG ATC AC-3'; 1370F, 5'-CGT CTC CGC TCG TCA CTT AT- 3', EGFP2, 5'-CTC GGC ATG GAC GAG CTG TA-3'; linker 1, 5'-GTA ATA CGA CTC ACT ATA GGG C-3'; linker 2, 5'-AGG GCT CCG CTT AAG GGA C-3'.

The sequences were aligned to the human genome (University of California, Santa Cruz, UCSC; hg18, March 2006, NCBI Build 36.1) using the BLAT program (http://genome.ucsc.edu/cgi-bin/hgBlat). A sequence was considered a genuine integration event if it contained both the 3'LTR from the nested primer to the end of 3'LTR (CA) and the linker sequence. Additionally, the sequence must match the human or mouse genome, respectively, with 95% or greater identity and the match must start immediately (within 3 bases) after the end of LTR sequence. Using these criteria, we mapped 468 integration sites (298 in the human and 170 in the mouse genome). All integration sites sequences have been deposited into GenBank under accession numbers: EU018613-EU019080. Comparison of integration targeting with other retroviruses was performed using data sets (298 randomly picked sites for each virus) downloaded from GenBank and mapped to the human genome. The following reports and GenBank accession numbers were used for the site selection: HTLV, (12), EF-580177-EF580913; ASLV, (28), CL528303-CL528772; HIV, (43), AY516881-AY517469; SIV, (10, 17), AY679815-AY680027, AY728482-AY728804; MLV, (43), AY515855-AY516880; FV (42), DU798511-DU799518. A set of 10,000 random integration sites in human and mouse genome, respectively, was generated and analyzed together with the integration sites of the retroviruses and was used as reference. Locations of the genomic features were downloaded from the UCSC genome database. 33 common insertion sites (CIS) commonly tagged in MMTV-induced mammary tumors determined by Theodorou (40) were used for comparison with the *de novo* integrations. Similarly, for comparison of the *in vitro* integrations with the more frequently tagged signalling pathways or protein domains the following data sets were used. FGF, TSP_1, PKINASE, CATION_ATPase, WNT, EGF, RAS, CYCLIN_C, GLA, IBN_N, TRUD for the frequently tagged Pfam protein domains, whilst MAPK signaling pathway, Wnt signaling pathway, Focal adhesion, Regulation of actin cytoskeleton, Calcium signaling pathway, Gap junction, Notch signaling pathway, Hedgehog signaling pathway for the KEGG pathways were used (40).

Determination of Kyoto Encyclopedia of Genes and Genomes (KEGG) pathways was performed using the US National Institutes of Health Database for Annotation, Visualization and Integrated Discovery (DAVID) software for KEGG (http://david.abcc.nciferf.gov/). Similarly, the Pfam protein domains represented in our data sets were identified using DAVID. Binomial distribution was used for calculation of the probability that a single integrant hits one of the 33 CIS that including a ±50kb region occupy 8.5×10⁶ bases of the mouse genome (29 × 10⁹ bases). P = n! / x! (n-x)! (p)^{x} (1-p)^{(n-x)}, where n equals the number of trials (170), x equals the number of hits (1) and p is probability of success on a single trial (8.5 × 10⁶ / 2.9 × 10⁹). Non-uniform distribution of CIS was ignored.

Phylogenetic trees were constructed by Neighbour-joining method using alignments of the amino acid sequences of the integrase proteins of MMTV, HTLV, ASLV, HIV, SIV, MLV and FV. Sequences were analyzed using the Clustal W 1.7 program (41) and software included in the PHYLIP package (14). Bootstrap analysis was made using 1000 replications.

### SHORT DESCRIPTION OF THE FIGURES

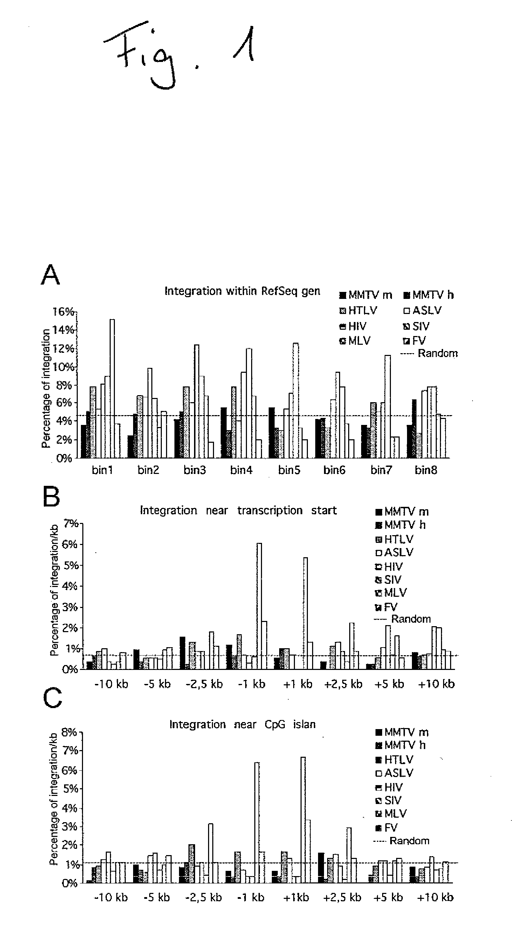

FIG. 1. Frequency of retroviral integration (MMTV in human [MMTV h] and mouse [MMTV m] genomes, HTLV, ASLV, HIV, SIV, MLV and FV) within and around transcription units, transcription start sites and CpG islands. A) Integration sites of the retroviruses were mapped relative to RefSeq transcription units divided into eight bins and plotted as the percentage of all integrations within each bin. B) Integration frequencies near transcription start sites of RefSeq genes. Percentage of all integrations per kb for each interval near the transcription start sites of RefSeq genes were plotted for each virus. C) Integration near CpG islands. Regions upstream and downstream of CpG islands were scored for the percentage of integrations per kb for each virus. The scattered line represents a mean random value calculated using the computer-generated random sites. Random value for the mouse genome is omitted.

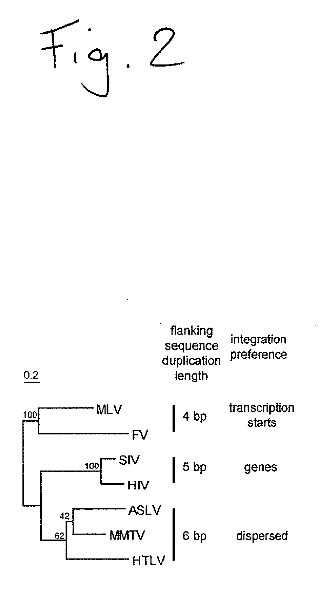

FIG. 2. Phylogenetic tree constructed by Neighbour-joining method based on an alignment of amino-acid sequences of the integrase proteins of MMTV, HTLV, ASLV, HIV, SIV, MLV and FV. Bootstrap values adjacent to each node represent percentage of 1000 trees supporting the clustering. The length of duplication generated by each virus during integration and preferential targeting of each virus is shown. The total branch length between the two species is proportional to the distance for each pair of species. Scale bar shows the distance.

### Results

Many genetic approaches that are routinely used with other retroviruses are complicated when they are applied to studies conducted with MMTV, mainly due to the poor replicative capacity of the virus *in vitro.* The titers obtained in cell culture are considerably lower than titers observed for other retroviruses, which makes analysis of MMTV integration targeting more difficult. Nevertheless, using linker-mediated PCR (LM-PCR) we could, on average, determine up to ten integration sites in a single round infection experiment. In order to develop a large data set suitable for statistical analyses and comparisons with the integration site specificities of other retroviruses we thus performed a series of single round infection experiments.

The second technical difficulty in working with MMTV is the presence of multiple endogenous MMTV sequences in the available susceptible mouse cell lines. MMTV specific primers used for the LM-PCR could thus hybridize with the endogenous sequences, which would then lead to the amplification of the endogenous segments instead of the *de novo* virus-host junctions. As the unique *de novo* junctions are underrepresented in the infected cells (one unique *de novo* integration compared to two to ten endogenous proviruses per cell in most inbred mouse strains (9)), one might expect that the endogenous sequences amplified in the LM-PCR would predominate over the *de novo* integrations. We circumvented this problem by using an infectious molecular clone of MMTV carrying the *EGFP* gene in the 3'LTR (MMTV-EGFP) (18). The presence of the *EGFP* gene on the integrated proviral sequence allowed us to use *EGFP*-specific primers thereby specifically amplifying only those virus-host junction sequences that resulted from the *de novo* infection events.

We used the recombinant MMTV-EGFP virus for infection of both the murine mammary cell line, NMuMG, as well as human breast cells, Hs578T. It had long been believed that human cells are not susceptible to MMTV, however our previous studies revealed that among others the human cell line Hs578T sustains MMTV infection and also supports replication of the virus (18). This cell line was infected with the MMTV-EGFP virus in single round infection experiments, in which the DNA was extracted from the infected cells 48h after infection. Additionally, as this human cell line does not contain endogenous MMTV sequences, we also infected these cells with a wild type MMTV (MMTV(GR)). In this case the infected cells were cultured in the presence of dexamethasone (10⁻⁶M DEX), to support replication of the virus, for four weeks. At the time of genomic DNA extraction, all of the cells in culture were infected, as could be demonstrated by an immunofluorescence staining of Gag proteins (data not shown). Genomic DNA, extracted either from the MMTV-EGFP- or MMTV(GR)-infected human cells was then submitted to LM-PCR as described previously (43). Sequences were trimmed and mapped to the human genome (UCSC; hg18, March 2006, NCBI Build 36.1). A total of 298 unique integration sites were mapped from the infected Hs578T cells. Of these, 82 and 216 integrations were cloned from the MMTV-EGFP- and MMTV(GR)-infected cells, respectively. Local features at each integration site were examined and the data sets obtained for both viruses, MMTV-EGFP and MMTV(GR), were compared. No statistically significant differences in any of the analyzed genomic features were found and so these two data sets were pooled and used for the further analyses reported here.

Murine cells, NMuMG, infected with MMTV-EGFP were analyzed in a similar manner. The genomic DNA was extracted 48h after the infection and amplified by LM-PCR. The PCR products were cloned and sequenced and the positions of the integration sites were determined using the mouse genome database (UCSC; mm8, Feb 2006, NCBI Build 36). A total of 170 unique integration sites were determined.

First, we sought to assess whether MMTV displays a tendency to integrate within transcription units by measuring the proportion of MMTV integrants within RNA polymerase II genes using human or mouse RefSeq genes (Table 1). This analysis revealed that of the 298 unique integrations mapped in the human genome, 105 (35.35%) occurred in the defined RefSeq gene set. Similarly, analysis of the integrations in the mouse genome showed that 55 of 170 (32.72%) integration events mapped within RefSeq genes (Table 1). When the integration preferences of MMTV determined in human and mouse genomes, respectively, were compared to the computer-generated random sites found in RefSeq, no significant difference was observed (35.92% and 34.99%, respectively) (Table 1).

Next, we compared the frequency of MMTV integration in transcription units observed in human cells with those of six other retroviruses (HIV, MLV, SIV, FV, ASLV, HTLV) using the same number (298 per virus) of randomly picked integrations, and to *the in silico-*generated random sites. As previously reported, the highest frequency of proviral integrations in transcription units, was seen for HIV (70.0%; P<0.0001) and SIV (75.1%; P<0.0001). MLV, ASLV and HTLV showed a modest, but significant, preference for integration within RefSeq genes (45.9%, 46%, 44.6%; P<0.0001). FV, in contrast, displayed no significant preference for RefSeq genes (28.0%) compared to random sites (35.0%). Similarly to FV, MMTV did not show any evidence for a tendency to integrate in RefSeq genes either in human or in mouse cells (Table 1).

The distribution of integration sites within the transcription units was also analysed. All transcription units were divided into eight bins, starting from the transcription start site. In agreement with previous analyzes, MLV integrations were preferentially located near the transcription start of genes, with 15,1 % of integrations found in the first bin (P<0.0001). HIV and SIV, the two viruses integrating preferentially into genes, tended to integrate in the middle of genes. A modestly higher frequency of integrations in the first four bins was found for HTLV. MMTV, like ASLV, showed roughly even distribution throughout all eight bins, irrespective of whether the integrations in the mouse or the human genome were analyzed (Fig. 1A).

Taken together, we conclude that independently of species, MMTV does not preferentially integrate within genes or any segment within transcription units.

Previously, it was reported that some retroviruses, such as MLV and to a lesser extent FV, favor integration in the vicinity of transcription start sites (42, 43). Thus, it was not surprising that, when the data sets obtained for MLV and FV were analyzed, the proportion of MLV (16.1%) and FV (6.0%) integrations located within ± 2kb of transcription start sites was significantly greater than expected for random integrations for both viruses (P<0.0001 and P<0.01, respectively). A weak bias in favor of transcription start sites was also seen for HTLV (5%, P<0.07) (Table 1, Fig. 1B). For MMTV, with 1.7% integrations found in human and 2.4% in mouse cells, respectively, no significant preference for transcription start sites compared to the computer-generated random sites (3.2% and 2.6%, respectively) was found. Similarly, no preference for ± 2kb of transcription start sites was observed for ASLV, HIV and SIV (3.0%, 2.0%, 0.6%, respectively) (Table 1, Fig. 1B).

CpG islands are thought to be associated with transcription initiation in vertebrates. These regions remain unmethylated in all cell types and often surround promoters of housekeeping genes (1). Studies of integration tendencies in CpG islands thus may not only reveal preferences of various retroviruses for integration near transcription start sites but also can possibly uncover an influence of DNA methylation on integration. Using data sets of the six previously analyzed retroviruses we were able to generate results which confirmed those found by others (10,12,28,29,42,43). Again, the strongest preference for integration near CpG islands was found for MLV with 20.1 % integrations within ± 2kb of CpG islands, followed by FV with 9.4% of integrants found in the same region. A weak preference for CpG islands was seen also for HTLV (7.7% in the ± 2kb window, P<0.01). All the other viruses (ASLV, HIV, SIV) including MMTV showed no preference for CpG islands compared to random data sets in both human as well as mouse cells (Table 1, Fig. 1C).

We also determined the frequency of integrations in repetitive elements, which account for nearly half of the human and mouse genome sequence, respectively. Of these, long interspersed nuclear elements (LINE), short interspersed nuclear elements (SINE) and LTR retrotransposons (LTR) are the most abundant. No tendency towards integration into these regions was determined for MMTV in either mouse or human cells (Table 1). Of note, among the analyzed retroviruses is the tendency of HTLV to avoid integrating in the most frequent repeats. A lower number of integrations (7.0%, P<0.01; 13.7%, P<0.01; 5%, P<0.05; respectively) compared to the *in silico*-generated integrants (12.6%, 19.6%, 8.1%, respectively) was observed for SINEs, LINEs as well as for LTRs, respectively. Additionally, a significantly greater number of the HTLV integrants were found in non repetitive compared to random sequences (68%, 54.8%, P<0.001, respectively). A similar trend was also seen for HIV and MLV (63%, P<0.01; 68.6%, P<0.001; respectively). A significantly decreased probability for integration within LINEs was also detected for MLV and FV (10.5%, P<0.001; 15.1%, P<0.05; respectively). ASLV, on the other hand, showed a modest trend in favor of integration into LINEs (24.3%, P<0.05) (Table 1).

Other repetitive elements such as DNA repeat elements, simple repeats (micro-satellites), low complexity repeats, satellite repeats, RNA repeats (including RNA, tRNA, rRNA, snRNA, scRNA) and other repeats, represent a minor portion of the overall genome and no significant number of MMTV integrations within any of these genomic segments could be observed (Table 1).

Recently, high-throughput screening of MMTV proviral insertion sites (664 independent virus-host flanking sequences) in 160 mammary tumors, arising after infection of BALB/c+ mice with C3H-MMTV, identified 33 common insertion sites (CIS) frequently targeted by MMTV in tumors (40). The availability of the database of the identified integration sites prompted us to investigate whether MMTV tends to integrate in the CIS identified in the above mentioned study. In tumor cells the most frequent genomic loci hosting MMTV proviral sequences belonged either to *Wnt* or *Fgf* genes (about 60% and 40% of tumors harbor the MMTV-tagged *Wnt* or *Fgf* genes) (9, 40). When the *de novo* MMTV integrations determined either in mouse or human genomes were screened for the presence of the integrants within ± 50kb of *Wnt* or *Fgf* genes, no such integration event was found (we included the ± 50kb gene flanking regions because enhancer-mediated activation is known to act over long distances (9)). Likewise, the other CIS identified in the study (40), were not tagged by MMTV after infection of human cells. One out of 170 proviral insertions identified in the mouse genome had integrated in *Odzl* gene, one of the CIS (Table 2). If we take into account the probability that one of the 170 integrations occurs in the vicinity of a CIS (including ± 50kb flanking regions occupy 8.4×10⁶ nt in the mouse genome) is 0.32, then such a result is not surprising and probably reflects random integration. One and two integrations in the human orthologues of mouse CIS were also identified for ASLV and HIV, respectively.

The recent study of Theodorou and colleagues also revealed that genes often targeted in mammary tumors belong to the same gene family or act in the same oncogenic pathway (40). Therefore, we sought to examine if some of the commonly tagged pathways or protein families were overrepresented in our data set. First, we identified all genes, including ± 50kb flanking regions, associated with MMTV integrations and then we determined any signaling cascades in which these MMTV-tagged genes might be involved using the US National Institutes of Health Database for Annotation, Visualization and Integrated Discovery (DAVID) software for Kyoto Encyclopedia of Genes and Genomes (KEGG) pathways (Supplemental material). In a similar way, we identified any Pfam protein domains (DAVID) present in our data set (Supplemental material). The protein domains and pathways were then compared to those that are significantly overrepresented in the MMTV-tagged mammary tumors (40). Data from the other six retroviruses as well as the random data sets were also analyzed. We could not detect any bias favoring the signaling pathways overrepresented in the MMTV-tagged tumors (Table 3). A modestly greater number of genes encoding proteins containing EGF protein domain were detected among those integration sites targeted by MMTV in the mouse genome when compared to random integrations (4.6% of the tagged genes encode protein with the EGF domain, P<0.05) (Table 4). One of these genes, *Odz1,* belongs to the CIS. The other genes (*3110045G13Rik, Celsr2, Ltbp1, Nrxn3, Selp, Vldlr*) have not previously been shown to host MMTV proviruses. Not even a weak preference for these groups (or any other group of genes) was found in the MMTV-infected human cells.

MMTV belongs to the Betaretrovirus genus, the only genus of the Retroviridae for which a large-scale mapping study of *de novo* retroviral integration sites has not been performed so far. In this study we investigated the integration site selection after infection of human and mouse mammary cells with MMTV. A total of 468 integration sites were obtained. Of these, 298 unique integration sites were determined from independent infection events in human cells, which further substantiate our previously published data that human cells are infectable with MMTV (18).

Since MMTV is a relatively "low titer virus" and since the mouse genome contains endogenous MMTV loci, we circumvented some of the difficulties in working with MMTV by performing a series of infection experiments using a recombinant MMTV-EGFP virus. In parallel, since human genome does not contain endogenous MMTV sequences, we infected the human breast cells with wild type MMTV(GR). In the presence of dexamethasone, a glucocorticoid stimulating the major MMTV promoter, the virus eventually infected all cells in culture (determined by immunofluorescence Gag imaging), thereby making the determination of the integration sites easier.

We sought to minimize any possible selection bias of integration sites by avoiding antibiotic selection of cell clones or extended growth of infected cells in cell culture. Comparisons of the integration sites targeted by MMTV-EGFP (harvested 48h after infection) and MMTV(GR), where further infections and superinfections are possible, did not reveal any obvious differences. Prior reports from others have also shown that short-term drug selection does not significantly influence the populations of recovered integration sites (21). We did not observe any prominent LM-PCR product that would reflect early integration events, lending credence to the idea that four weeks cultivation time is not long enough for significant clonal expansion of early infectants.

The integration profile determined after MMTV infection of human cells resembled that in mouse cells. In both cases we did not observe preferential targeting of genes, in contrast to what has previously been shown for HIV and SIV. Likewise, we have not seen integration favoring transcription start sites as has been reported for MLV and FV. We did find, on the other hand, that MMTV, irrespective whether the virus infects mouse or human cells, appears to integrate randomly, as has been shown for ASLV and HTLV. Random dispersion of the integration events in both species suggests that either there is no cellular factor(s) that interacts with the MMTV preintegration complex (PIC) and helps to tether this complex to the chromosomal DNA or, more likely, potential cellular factor(s) are conserved between mice and humans. Another possible reason for random dispersion of the MMTV integration sites might also be the lack of expression of the cellular factor(s) in the cell lines used in the study. Although it cannot be ruled out, it does seem rather unlikely that the preintegration complex-interacting partner(s) would be absent in specifically these two cell lines. We have selected mouse and human mammary cell lines because mammary epithelial cells are major targets during *in vivo* infection.

Not much is known about cellular factors interacting with PICs, especially with the PIC of MMTV. Recently, it was shown that LEDGF/p75 binds to HIV integrase and plays a role in HIV integration site selection (6, 22, 23). Another cellular protein that interacts with the PIC is BAF. This protein, which, in contrast to LEDGF/p75, binds to MLV as well as to HIV PICs, is known to be a factor that blocks the self-destructive integration of proviral DNA into its own genome *in vitro* (5, 20, 36). Recently, it was also shown that BAF, together with its own binding partner the nuclear-envelope-associated protein, Emerin, is required for the appropriate localization of HIV cDNA before chromatin engagement. In contrast to HIV, Emerin is not required for the appropriate localization of MLV cDNA before chromatin tethering (19). The PIC of MLV rather interacts with other member of the LEM family of inner nuclear membrane and nucleoplasmic proteins such as LAP2a (38). Further work is needed to uncover whether such proteins also interact with PICs of viruses displaying random distribution of integration sites.

MMTV, analogously to ASLV and HTLV, generates 6bp-long duplications of host sequence flanking the integrated proviruses (25). Additionally, Derse and coworkers suggested that the integration profile of various retroviruses could be predicted from the phylogenetic analysis of retroviral integrases (12).

In such a phylogenetic analysis the integrase of MMTV is clustered in a branch comprising integrases of ASLV, and HTLV (Fig. 2), two retroviruses that display little integration preference for any genomic feature, providing a basis for the prediction that the integration profile of MMTV will resemble that of ASLV, and HTLV. In accordance with this prediction we observed that the integration site preference of MMTV most closely resembles that of ASLV, and HTLV. However, we did not observe any preference for integration within genes. Whereas 44.8% and 46% of the integrated HTLV and ASLV, proviruses, respectively, were found within genes, only 32.7% and 35.4% of the MMTV integrants in mouse and human cells, respectively, landed within RefSeq regions, a frequency that is not significantly different film that of the computer-generated random sites. Additionally, unlike for ASLV and HTLV, we did not see a significant difference in frequency of MMTV integration in repetitive elements compared with random sites. HTLV also does not preferentially integrate into LINEs, SINEs and LTR retrotransposons, whereas ASLV shows a slight bias in favor of LINEs. Taken together, it appears that MMTV displays the most random integration site distribution among retroviruses determined to date.

MLV-derived vectors are currently the most widely used vectors in clinical gene transfer protocols. Clinical trials with MLV-based vectors revealed that, under certain circumstances, instertional mutagenesis of *LMO2* gene led to the development of leukemia in some of the treated X-linked severe combined immune deficiency patients (reviewed in (4)). Taking into account the random integration targeting exhibited by MMTV, it can be speculated that construction of a hybrid vector carrying the MMTV integrase would improve safety. Integrase was previously demonstrated to be a major determinant of retroviral insertion targeting and a hybrid HIV carrying the integrase of MLV, targeted sites with a specificity close to that of MLV (21). Obviously, this concept would require further studies to elucidate whether the MMTV integrase alone would be sufficient for random site selection or whether other factors that are known to participate in the integration reaction such as Gag and terminal LTR sequences are needed. Concerns regarding the safety of such vectors could also be raised due to the fact that MMTV is known as an insertional mutagen. However, we have not observed an increased integration targeting of the genes previously described as common insertion sites often tagged by MMTV in mammary tumors. Likewise, we have not seen a significant bias in favor of genes encoding proteins containing domains or involved in signaling pathways commonly found in MMTV-induced tumors. Thus, it may be possible to improve the safety of gene therapy vectors by using an integrase which would not direct proviral DNA to the vicinity of or within genes. The integrase of MMTV, the virus showing the most random distribution of integration sites yet, might be a good option for such an improvement.

**TABLE 1. Integration frequency near genomic features**

| Location^{a} | Frequency of integration (%) | | | | | | | | | |
|---|---|---|---|---|---|---|---|---|---|---|
| | Mouse genome | | Human genome | | | | | | | |
| | random sites | MMTV | random Sites sites | MMTV | HTLV | ASLV | HIV | SIV | MLV | FV |
| RefSeq | 34.99 | 32.72 | 35.92 | 35.35 | 44.60** | 46.00*** | 70.03**** | 75.14**** | 45.90*** | 28.00** |
| ±2 kb TSS | 2.64 | 2.42 | 3.20 | 1.68 | 5.00 | 3.00 | 2.02 | 0.60 | 16.11**** | 6.00** |
| ±2 kb CpG | 2.82 | 4.85 | 4.18 | 2.01 | 7.67** | 6.30 | 3.36 | 1.20 | 20.14**** | 9.36**** |
| SINE | 7.34 | 8.28 | 12.55 | 10.40 | 7.00** | 11.00 | 15.49 | 16.67 | 10.81 | 11.71 |
| LINE | 20.08 | 14.79 | 19.63 | 21.81 | 13.67** | 24.33* | 15.49 | 16.67 | 10.47*** | 15.05* |
| LTR | 9.89 | 14.20 | 8.07 | 7.72 | 5.00* | 9.00 | 4.38* | 7.14 | 5.74 | 9.36 |
| rest | 4.45 | 3.55 | 4.98 | 4.03 | 6.33 | 2.33 | 1.68 | 6.55 | 4.39 | 7.02 |
| no repeat | 58.24 | 59.17 | 54.77 | 56.04 | 68.00*** | 53.33 | 62.96 | 52.98 | 68.58*** | 56.86 |

| | | | | | | | | | | |
|---|---|---|---|---|---|---|---|---|---|---|
| P values for chi-square comparison to random integrations: *P< 0.05,** P<0.01, *** P<0.001, **** P<0.0001 ^{a}Abbreviations: TSS, transcription start site; CpG, CpG island; SINE, short interspersed nuclear element; LINE, long interspersed nuclear element; LTR, LTR retrotransposons; rest, includes DNA repeat elements, simple repeats (micro-satellites), low complexity repeats, satellite repeats, RNA repeats (including RNA, TRNA, rRNA, snRNA, seRNA) | | | | | | | | | | |

**TABLE 2. de novo MMTV integrants found in common integration sites (CIS) determined in MMTV-induced tumors^{a}**

| Virus / random sites | Gene^{d} |
|---|---|
| random sites (mouse)^{b} | - |
| MMTV (mouse) | *Odz1* |
| random sites (human)^{c} | - |
| MMTV (human) | - |
| HTLV | - |
| ASLV | *FGF8* |
| HIV | *ATP2B1, LAMB1* |
| SIV | - |
| MLV | - |
| FV | - |

| | |
|---|---|
| ^{a}33 CIS were determined by Theodorou (40) ^{b}170 computer-generated random sites ^{c}298 computer-generated random sites ^{d}genes including ±50 kb flanking regions targeted by a virus | |

**TABLE 3. Frequency of de novo integrations in genes that belong to commonly tagged KEGG pathways in MMTV.induced mammary tumors**

| KEGG pathway (NIH-DAVID)^{b} | Frequency of integration (%)^{a} | | | | | | | | | |
|---|---|---|---|---|---|---|---|---|---|---|
| | Mouse genome | | Human genome | | | | | | | |
| | random sites | MMTV | random sites | MMTV | HTLV | ASLV | HIV | SIV | MLV | FV |
| MAPK signaling pathway | 1.58 | 0.66 | 1.62 | 0.77 | 2.88 | 2.57 | 1.30 | 2.72 | 1.59 | 1.12 |
| Wnt signaling pathway | 0.89 | 1.32 | 0.88 | 1.15 | 0.64 | 0.29 | 1.08 | 1.26 | 1.85 | 1.12 |
| Focal adhesion | 1.37 | 0.66 | 1.40 | 1.54 | 1.60 | 0 | 1.30 | 0.84 | 2.12 | 2.99 |
| Regulation of acitn | | | | | | | | | | |
| cytoskeleton | 1.27 | 0.66 | 1.27 | 1.54 | 1.60 | 2,86 | 1.51 | 1.67 | 1.59 | 1.12 |
| Calcium signaling pathway | 1.23 | 1.97 | 1.40 | 1.54 | 0.96 | 0.86 | 1.08 | 1.46 | 1.32 | 1.12 |
| Gap junction | 0.60 | 0 | 0.66 | 1.15 | 1.28 | 0.86 | 0.43 | 1.05 | 1.32 | 1.12 |
| Notch signaling pathway | 0.22 | 0 | 0.25 | 0.38 | 0 | 0.29 | 0.65 | 0.42 | 0.26 | 0 |
| Hedgehog signaling pathway | 0.32 | 0 | 0.35 | 0 | 0.64 | 0 | 0.86 | 0 | 0.53 | 0 |

| | | | | | | | | | | |
|---|---|---|---|---|---|---|---|---|---|---|
| ^{a}% from total number of genes (including ±50 kb region) targeted by virus or random sites ^{b}KEGG pathways commonly targeted in tumors as determined by Theodorou (40) | | | | | | | | | | |

**TABLE 4. Frequency of de novo integrations in genes encoding proteins with Pfam protein domains common tagged in MMTV-induced mammary tumors**

| Pfam protein domain name (NIH-DAVID)^{b} | Frequency of integration (%)^{a} | | | | | | | | | |
|---|---|---|---|---|---|---|---|---|---|---|
| | Mouse genome | | Human genome | | | | | | | |
| | random sites | MMTV | random sites | MMTV | HTLV | ASLV | HIV | SIV | MLV | FV |
| FGF | 0.10 | 0 | 0.09 | 0 | 0 | 0.29 | 0 | 0 | 0.26 | 0.37 |
| TSP_1 | 0.38 | 0 | 0.53 | 0.38 | 0.32 | 0.29 | 0.22 | 0 | 0.26 | 0.75 |
| PKINASE | 2.23 | 1.32 | 2.37 | 1.92 | 2.24 | 3.14 | 4.75* | 2.30 | 3.44 | 1.87 |
| CATION_ATPase | 0.06 | 0.66 | 0.09 | 0 | 0 | 0 | 0.43 | 0.21 | 0 | 0 |
| WNT | 0.14 | 0 | 0.13 | 0 | 0 | 0 | 0.22 | 0 | 0 | 0 |
| EGF | 0.94 | 4.61* | 0.94 | 1.92 | 1.28 | 1.43 | 0.65 | 0.42 | 1.59 | 1.12 |
| RAS | 0.68 | 0.66 | 0.58 | 0.77 | 0.64 | 0.86 | 0.43 | 0.63 | 0.79 | 0.37 |
| CYCLIN_C | 0.11 | 0 | 0.05 | 0 | 0 | 0 | 0 | 0 | 0.26 | 0 |
| GLA | 0.03 | 0 | 0.09 | 0 | 0 | 0.29 | 0 | 0 | 0.53 | 0 |
| IBN_N | 0.10 | 0 | 0.16 | 0 | 0 | 0.29 | 0.22 | 0.21 | 0 | 0 |
| TRUD | 0 | 0 | 0.02 | 0 | 0 | 0 | 0 | 0 | 0 | 0 |

| | | | | | | | | | | |
|---|---|---|---|---|---|---|---|---|---|---|
| *P values for chi-square comparison to random integrations, *P<0.05 ^{a}% from total number of genes (including ±50 kb region) targeted by virus or random sites ^{b} Pfam protein domains commonly targeted in tumors as determined by Theodorou (40) | | | | | | | | | | |

### References

1. Alberts, A., A. Johnson, J. Lewis, M. Raff, K. Roberts, and P. Walter. 2002. Molecular Biology of the Cell, 4th ed. Garland Science.
2. Barr, S. D., J. Leipzig, P. Shinn, J. R. Ecker, and F. D. Bushman. 2005. Integration targeting by avian sarcoma-leukosis virus and human immunodeficiency virus in the chicken genome. J Virol 79:12035-44.
3. Bittner, J. J. 1936. Some possible effects of nursing on the mammary gland tumor incidence in mice. Science 84:162.
4. Bushman, F. D. 2007. Retroviral integration and human gene therapy. J Clin Invest 117:2083-6.
5. Chen, H., and A. Engelman. 1998. The barrier-to-autointegration protein is a host factor for HIV type 1 integration. Proc Natl Acad Sci U S A 95:15270-4.
6. Ciuffi, A., M. Llano, E. Poeschla, C. Hoffmann, J. Leipzig, P. Shinn, J. R. Ecker, and F. Bushman. 2005. A role for LEDGF/p75 in targeting HIV DNA integration. Nat Med 11:1287-9.
7. Clausse, N., D. Baines, R. Moore, S. Brookes, C. Dickson, and G. Peters. 1993. Activation of both Wnt-1 and Fgf-3 by insertion of mouse mammary tumor virus downstream in the reverse orientation: a reappraisal of the enhancer insertion model. Virology 194:157-65.
8. Clausse, N., R. Smith, C. M. Calberg-Bacq, G. Peters, and C. Dickson. 1993. Mouse mammary-tumor virus activates Fgf-3/Int-2 less frequently in tumors from virgin than from parous mice. Int J Cancer 55:157-63.
9. Coffin, J. M., S. H. Hughes, and H. E. Varmus. 1997. Retroviruses. Cold Spring Harbor Laboratory Press.
10. Crise, B., Y. Li, C. Yuan, D. R. Morcock, D. Whitby, D. J. Munroe, L. O. Arthur, and X. Wu. 2005. Simian immunodeficiency virus integration preference is similar to that of human immunodeficiency virus type 1. J Virol 79:12199-204.
11. Dekaban, G. A., and J. K. Ball. 1984. Integration of type B retroviral DNA in virus-induced primary murine thymic lymphomas. J Virol 52:784-92.
12. Derse, D., B. Crise, Y. Li, G. Princler, N. Lum, C. Stewart, C. F. McGrath, S. H. Hughes, D.J. Munroe, and X. Wu. 2007. HTLV-1 integration target sites in the human genome: comparison with other retroviruses. J Virol.
13. Dudley, J., and R. Risser. 1984. Amplification and novel locations of endogenous mouse mammary tumor virus genomes in mouse T-cell lymphomas. J Virol 49:92-101.
14. Felsenstein, J. 1989. PHYLIP - Phylogeny Inference Package (Version 3.2). Cladistics 5:164-166.
15. Gallahan, D., and R. Callahan. 1987. Mammary tumorigenesis in feral mice: identification of a new int locus in mouse mammary tumor virus (Czech II)-induced mammary tumors. J Virol 61:66-74.
16. Gray, D. A., C. M. McGrath, R. F. Jones, and V. L. Morris. 1986. A common mouse mammary tumor virus integration site in chemically induced precancerous mammary hyperplasias. Virology 148:360-8.
17. Hematti, P., B. K. Hong, C. Ferguson, R. Adler, H. Hanawa, S. Sellers, I. E. Holt, C. E. Eckfeldt, Y. Sharma, M. Schmidt, C. von Kalle, D. A. Persons, E. M. Billings, C. M. Verfaillie, A. W. Nienhuis, T. G. Wolfsberg, C. E. Dunbar, and B. Calmels. 2004. Distinct genomic integration of MLV and SIV vectors in primate hematopoietic stem and progenitor cells. PLoS Biol 2:e423.
18. Indik, S., W. H. Gunzburg, B. Salmons, and F. Rouault. 2005. Mouse mammary tumor virus infects human cells. Cancer Res 65:6651-9.
19. Jacque, J. M., and M. Stevenson. 2006. The inner-nuclear-envelope protein emerin regulates HIV-1 infectivity. Nature 441:641-5.
20. Lee, M. S., and R. Craigie. 1994. Protection of retroviral DNA from autointegration: involvement of a cellular factor. Proc Natl Acad Sci U S A 91:9823-7.
21. Lewinski, M. K., M. Yamashita, M. Emerman, A. Ciuffi, H. Marshall, G. Crawford, F. Collins, P. Shinn, J. Leipzig, S. Hannenhalli, C. C. Berry, J. R. Ecker, and F. D. Bushman. 2006. Retroviral DNA integration: viral and cellular determinants of target-site selection. PLoS Pathog 2:e60.
22. Llano, M., D. T. Saenz, A. Meehan, P. Wongthida, M. Peretz, W. H. Walker, W. Teo, and E. M. Poeschla. 2006. An essential role for LEDGF/p75 in HIV integration. Science 314:461-4.
23. Llano, M., M. Vanegas, O. Fregoso, D. Saenz, S. Chung, M. Peretz, and E. M. Poeschla. 2004. LEDGF/p75 determines cellular trafficking of diverse lentiviral but not murine oncoretroviral integrase proteins and is a component of functional lentiviral preintegration complexes. J Virol 78:9524-37.
24. MacNeil, A., J. L. Sankale, S. T. Meloni, A. D. Sarr, S. Mboup, and P. Kanki. 2006. Genomic sites of human immunodeficiency virus type 2 (HIV-2) integration: similarities to HIV-1 in vitro and possible differences in vivo. J Virol 80:7316-21.
25. Majors, J. E., and H. E. Varmus. 1981. Nucleotide sequences at host-proviral junctions for mouse mammary tumour virus. Nature 289:253-8.
26. Marchetti, A., F. Buttitta, S. Miyazaki, D. Gallahan, G. H. Smith, and R. Callahan. 1995. Int-6, a highly conserved, widely expressed gene, is mutated by mouse mammary tumor virus in mammary preneoplasia. J Virol 69:1932-8.
27. Michalides, R., E. Wagenaar, J. Hilkens, J. Hilgers, B. Groner, and N. E. Hynes. 1982. Acquisition of proviral DNA of mouse mammary tumor virus in thymic leukemia cells from GR mice. J Virol 43:819-29.
28. Mitchell, R. S., B. F. Beitzel, A. R. Schroder, P. Shinn, H. Chen, C. C. Berry, J. R. Ecker, and F. D. Bushman. 2004. Retroviral DNA Integration: ASLV, HIV, and MLV Show Distinct Target Site Preferences. PLoS Biol 2:E234.
29. Narezkina, A., K. D. Taganov, S. Litwin, R. Stoyanova, J. Hayashi, C. Seeger, A. M. Skalka, and R. A. Katz. 2004. Genome-wide analyses of avian sarcoma virus integration sites. J Virol 78:11656-63.
30. Nusse, R., and H. E. Varmus. 1982. Many tumors induced by the mouse mammary tumor virus contain a provirus integrated in the same region of the host genome. Cell 31:99-109.
31. Peters, G., S. Brookes, R. Smith, and C. Dickson. 1983. Tumorigenesis by mouse mammary tumor virus: evidence for a common region for provirus integration in mammary tumors. Cell 33:369-77.
32. Peters, G., C. Kozak, and C. Dickson. 1984. Mouse mammary tumor virus integration regions int-1 and int-2 map on different mouse chromosomes. Mol Cell Biol 4:375-8.
33. Roelink, H., E. Wagenaar, and R. Nusse. 1992. Amplification and proviral activation of several Wnt genes during progression and clonal variation of mouse mammary tumors. Oncogene 7:487-92.
34. Schroder, A. R., P. Shinn, H. Chen, C. Berry, J. R. Ecker, and F. Bushman. 2002. HIV-1 integration in the human genome favors active genes and local hotspots. Cell 110:521-9.
35. Schuermann, M., and R. Michalides. 1987. A rare common integration site of proviruses of the mouse mammary tumor virus in P-type mammary tumors of mouse strain GR. Virology 156:229-37.
36. Segura-Totten, M., and K. L. Wilson. 2004. BAF: roles in chromatin, nuclear structure and retrovirus integration. Trends Cell Biol 14:261-6.
37. Shackleford, G. M., C. A. MacArthur, H. C. Kwan, and H. E. Varmus. 1993. Mouse mammary tumor virus infection accelerates mammary carcinogenesis in Wnt-1 transgenic mice by insertional activation of int-2/Fgf-3 and hst/Fgf-4. Proc Natl Acad Sci U S A 90:740-4.
38. Suzuki, Y., H. Yang, and R. Craigie. 2004. LAP2alpha and BAF collaborate to organize the Moloney murine leukemia virus preintegration complex. Embo J 23:4670-8.
39. Theodorou, V., M. Boer, B. Weigelt, J. Jonkers, M. van der Valk, and J. Hilkens. 2004. Fgf10 is an oncogene activated by MMTV insertional mutagenesis in mouse mammary tumors and overexpressed in a subset of human breast carcinomas. Oncogene 23:6047-55.
40. Theodorou, V., M. A. Kimm, M. Boer, L. Wessels, W. Theelen, J. Jonkers, and J. Hilkens. 2007. MMTV insertional mutagenesis identifies genes, gene families and pathways involved in mammary cancer. Nat Genet.
41. Thompson, J. D., D. G. Higgins, and T. J. Gibson. 1994. CLUSTAL W: improving the sensitivity of progressive multiple sequence alignment through sequence weighting, position-specific gap penalties and weight matrix choice. Nucleic Acids Res 22:4673-80.
42. Trobridge, G. D., D. G. Miller, M. A. Jacobs, J. M. Allen, H. P. Kiem, R. Kaul, and D. W. Russell. 2006. Foamy virus vector integration sites in normal human cells. Proc Natl Acad Sci U S A 103:1498-503.
43. Wu, X., Y. Li, B. Crise, and S. M. Burgess. 2003. Transcription start regions in the human genome are favored targets for MLV integration. Science 300:1749-51.

## Claims

1. A viral vector comprising within its genome one or more heterologous coding and/or heterologous non-coding nucleic acid sequences, wherein the nucleic acid sequence coding for the retroviral integrase of said retroviral vector is replaced by a nucleic acid sequence coding for a Betaretrovirus integrase.

2. The viral vector according to claim 1, wherein the viral vector is a alpharetroviral vector, a gammaretroviral vector, a lentiretroviral vector or a deltaretroviral vector

3. The viral vector according to claim 1 or 2, wherein the Betaretrovirus integrase is Mouse Mammary Tumor Virus integrase.

4. The viral vector according to claims 1 to 3, wherein the viral vector is a replication incompetent viral vector.

5. A viral particle containing the viral vector according to anyone of claims 1 to 4.

6. A method for introducing heterologous nucleic acid sequences into target cells comprising infecting the target cell with the viral particles according to claim 5

7. A pharmaceutical composition containing a therapeutically effective amount of the viral vector according to any of claims 1-4 or/and the viral particle according to claim 5

8. Use of the viral vector according to any of the preceding claims 1 to 4 and/or of the viral particle according to claim 5 for producing a pharmaceutical composition for gene therapy.
